# EUROPEAN PATENT SPECIFICATION

(11) **EP 3 801 275 B1**
(45) Date of publication and mention of the grant of the patent: **08.10.2025**
(21) Application number: 18922057.7
(22) Date of filing: 07.06.2018
(51) Int. Cl.: A61B 8/00, A61B 5/026

(54) **APPARATUS AND METHOD FOR DIAGNOSING VESSEL OCCLUSION**
VORRICHTUNG UND VERFAHREN ZUR DIAGNOSE VON GEFÄSSVERSCHLÜSSEN
APPAREIL ET MÉTHODE POUR DIAGNOSTIQUER UNE OCCLUSION DE VAISSEAUX

(43) Date of publication of application: 14.04.2021
(73) Proprietor: Loh, Yince, Seattle, WA 98199 (US)
(72) Inventor: Loh, Yince, Seattle, WA 98199 (US)
(74) Representative: Murgitroyd & Company
(86) International application number: PCT/US2018/036525
(87) International publication number: WO 2019/236092

(56) References cited:
- US-A1- 2010 160 779
- US-A1- 2017 188 992

## Description

### CROSS-REFERENCES TO RELATED APPLICATIONS

This application claims priority to US Provisional Application No. 62486177, filed April 17, 2017, and US Provisional Application No. 62517459, dated June 9, 2017,.

### TECHNICAL FIELD

The present invention relates to a head-mounted diagnostic tool for diagnosing conditions consistent with the existence of blockage of a patient's cranial blood vessels, such as large vessel occlusions.

### BACKGROUND

Nearly 800,000 strokes occur in the US annually, and almost 3 million Americans are currently disabled from them. Stroke is the third leading cause of death in the US and is the leading cause of disability costing over $73 billion/year in the US alone.

The most disabling and deadly ischemic strokes (i.e. lack of blood flow to the brain) result from large vessel occlusions (LVO's). Patients with LVO's have extremely poor outcomes without treatment and until recently, respond poorly to standard of care (tissue plasminogen activator, or tPA). In the 1990s, the MERCI retrieval system marked the advent of an endovascular method and system that could be used to remove clot from the brain vessels. Several decades later, the second and third generation devices furthered the concept that clot in the brain vessels could be extracted using devices and catheters inserted through the groin artery. Within one year, five randomized controlled trials all showed a positive benefit of endovascular therapy (EVT) over optimal medical management of LVO's. All had a time limit for inclusion in the study and several showed that earlier intervention produced better clinical outcomes.

In one of these studies, patients transferred to a hospital without EVT capability had an average delay of two hours before arriving to the final EVT-capable facility. This is an unacceptable delay when time is critical to preserving brain function. State health departments, National Accreditation Organizations, and systems of care designers have implemented designations for stroke capabilities to distinguish those capable of providing standard of non- EVT stroke care and those with 24/7 EVT capability. Emergency medical systems (EMS) will be integral in appropriate patient triage and delivery to stroke centers, much like trauma triage. The emerging dilemma now lies in accurate field stroke triage. Only a portion of ischemic strokes result from LVO's, and EVT does not benefit the rest. Movement of both LVO and non-LVO stroke patients to a single EVT-capable center would potentially delay or deprive a patient of standard of care treatment for non-LVO strokes. It would potentially also overwhelm the EVT-capable hospital.

Imaging identification of LVO's already exists with MRI and CT. The former is not feasible for field deployment, while the field-deployable versions of the latter are extremely expensive and likely to be a limited yet paradoxically under-utilized resource. Transcranial Doppler (TCD) ultrasonography and near infrared scanners are portable tools that can identify LVO's, but are operator-dependent.

There is a need to diagnose LVQs quickly and provide appropriate medical intervention. The ideal adjunct to the EMT or paramedic assessing a possible stroke patient is a field-expedient, operator-independent device to help determine whether a patient potentially needs EVT. Such a device could effectively diagnose while minimizing diagnostic error and operator training. Such a device could also help emergency physicians at non-EVT hospitals identify EVT-eligible patients earlier and expedite transfer to EVT-capable hospitals without doing additional time-consuming imaging.

US 2017/188992 A1 discloses systems and methods for detecting neurological conditions utilising transcranial Doppler ultrasonography and US 2010/160779 A1 discloses an ultrasound diagnostic imaging system for cerebral blood flow imaging and microbubble-enhanced blood clot lysis.

### SUMMARY

An objective of this invention is to provide apparatus for diagnosing conditions consistent with the presence of blockage in a patient's cranial blood vessels, including the presence of LYO's, using the framework of a small head-harness that is transportable, field- expedient, and durable. A single pulse set using ultrasonic or near-infrared energy is broadcast into the patient's brain allowing the apparatus to perform an area scan of the brain and detect and decipher cranial blood vessel blockage and LVO signal patterns. The interpretation of the pattern lies within the internal programming which produces a binary signal as to whether an LVO is suspected.

According to the present invention, there is provided a head-mounted diagnostic tool as defined in claim 1. The embodiments discussed in this disclosure are for illustrative purposes, the invention being defined only by the appended claims.

### DRAWINGS DESCRIPTION

Other features and advantages of the present invention will become apparent in the following detailed descriptions of certain preferred embodiments with reference to the accompanying drawings, of which:

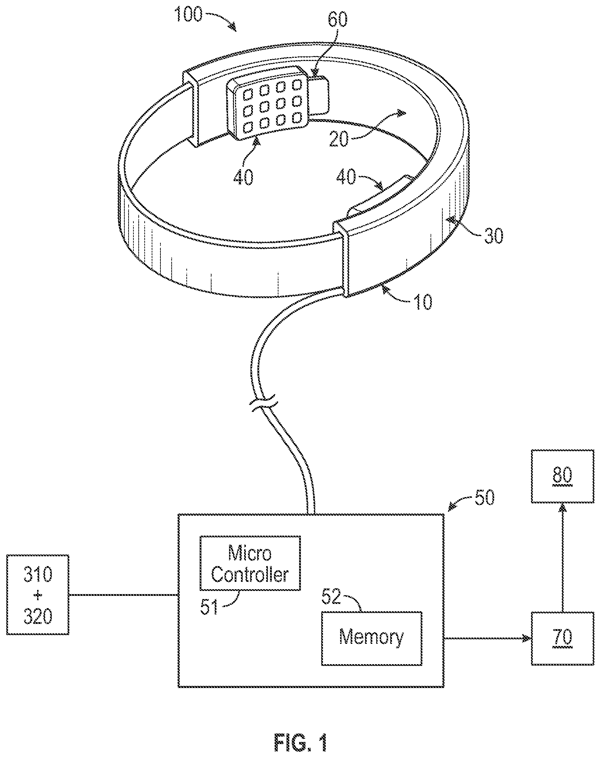

Fig. 1 is an elevation view of a small, lightweight, self-contained, portable, ruggedized, head-mounted diagnostic tool with a transducer array and other system components useful for diagnosing conditions consistent with the existence of large vessel occlusion according to illustrative embodiments of the present invention;

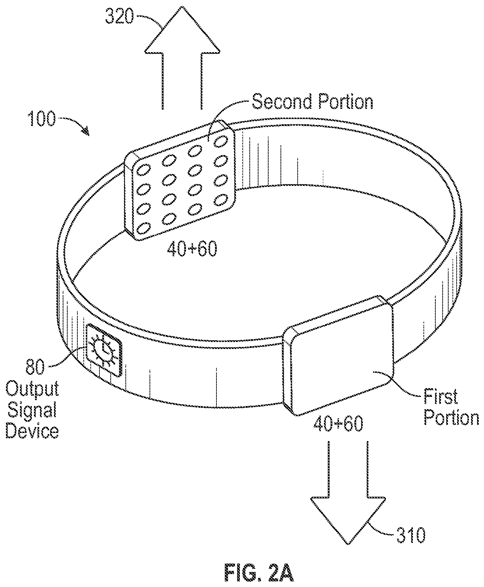

Fig. 2A is an elevation view of a small, lightweight, self-contained, portable, ruggedized, head-mounted diagnostic tool with a transducer array and other system components useful for diagnosing conditions consistent with the existence of large vessel occlusion according to illustrative embodiments of the present invention;

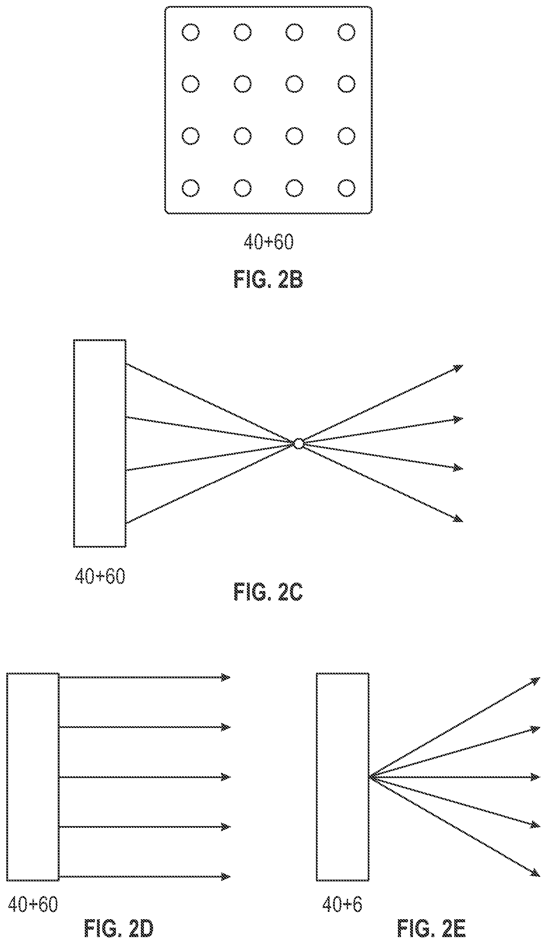

Fig. 2B is a plan view of a transducer array useful for diagnosing conditions consistent with the existence of large vessel occlusion according to illustrative embodiments of the present invention;
Fig. 2C is a side view of a transducer array illustrating one example of how energy from the transducer array is transmitted into a patient's brain using a single pulse set according to illustrative embodiments of the present invention;
Fig. 2D is a side view of a transducer array illustrating one example of how energy from the transducer array is transmitted into a patient's brain using a single pulse set according to illustrative embodiments of the present invention;
Fig. 2E is a side view' of a transducer array illustrating one example of how energy from the transducer array is transmitted into a patient's brain using a single pulse set according to illustrative embodiments of the present invention;

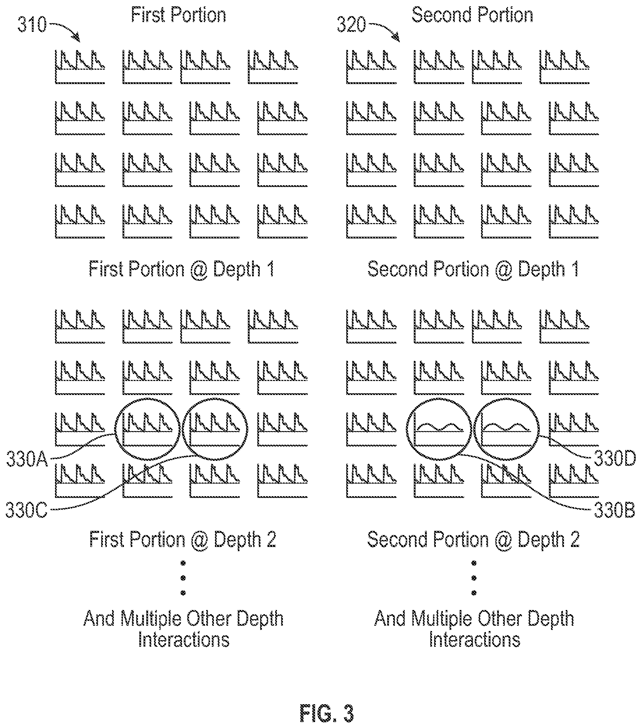

Fig. 3 is an illustration of one example of how data collected from a first portion of the brain is compared to that collected from a second portion of the brain wherein sufficient differences are identified to suggest the presence of LVO's in the patient's brain according to illustrative embodiments of the present invention;

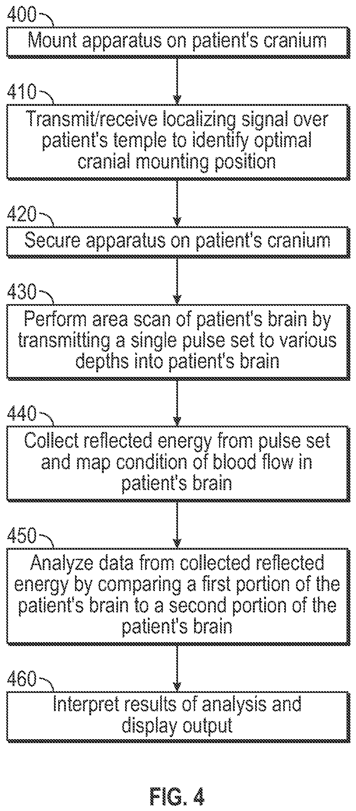

Fig. 4 show's a method of diagnosing conditions consistent with the existence of large vessel occlusion by performing an area scan of a patient's brain and comparing data collected from a first portion of a patient's brain with that collected from a second portion of a patient's brain using apparatus according to illustrative embodiments of the present invention;

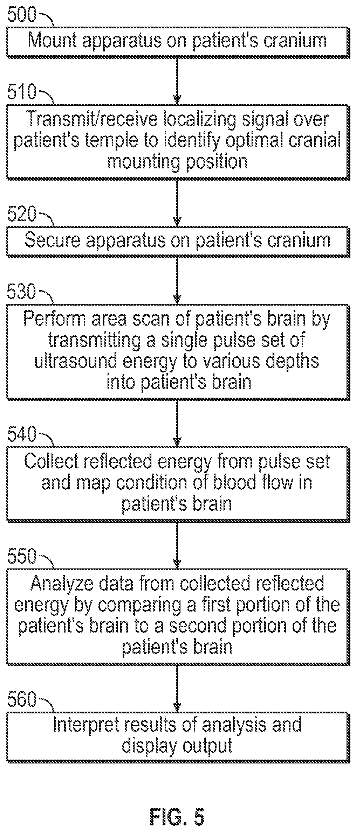

Fig. 5 shows a method of diagnosing conditions consistent with the existence of large vessel occlusion by performing an area scan of a patient's brain using transcranial ultrasound and comparing data collected from a first portion of a patient's brain with that collected from a second portion of a patient's brain using apparatus described but not claimed;

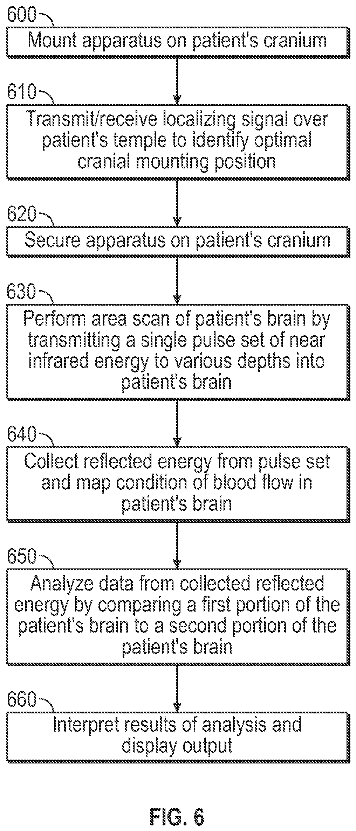

Fig. 6 shows a method of diagnosing conditions consistent with the existence of large vessel occlusion by performing an area scan of a patient's brain using near infrared imaging and comparing data collected front a first portion of a patient's brain with that collected from a second portion of a patient's brain using apparatus according to illustrative embodiments of the present invention; and

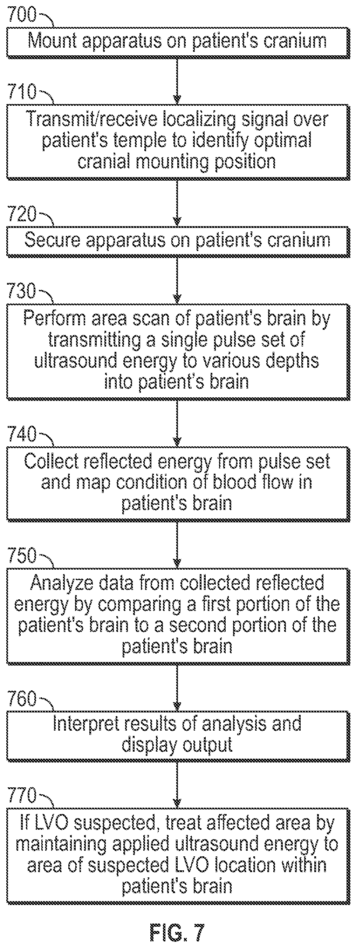

Fig. 7 shows a method of diagnosing conditions consistent with the existence of large vessel occlusion by performing an area scan of a patient's brain using transcranial ultrasound, comparing data collected from a first portion of a patient's brain with that collected from a second portion of a patient's brain, and treating suspected LVO using transcranial doppler energy, using apparatus described but not claimed.

### DESCRIPTION

In the Background, Summary, and Drawings Description above, in the Description and the claims below, and in the accompanying drawings, reference is made to particular features (including method steps) of the invention.

The term "comprises" and grammatical equivalents thereof are used herein to mean that other components, ingredients, steps, etc. are optionally present. For example, an article "comprising" (or "which comprises") components A, B, and C can consist of (i.e. contain only) components A B, and C, or can contain not only components A, B and C but also one or more other components.

Where reference is made herein to a method comprising two or more defined steps, the defined steps can be carried out in any order or simultaneously (except where the context excludes that possibility), and the method can include one or more other steps which are carried out before any of the defined steps, between two of the defined steps, or after all the defined steps (except where context excludes that possibility).

The term "at least" followed by a number is used herein to denote the start of a range beginning with that number (which may be a range having an upper limit or no upper limit, depending on the variable being defined). For example "at least 1" means 1 or more than 1. The term "at most" followed by a number is used herein to denote the end of a range ending with that number (which may be a range having 1 or 0 as its lower limit, or a range having no lower limit, depending upon the variable being defined). For example "at most 4" means 4 or less than 4, and "at most 40%" means 40% or less than 40%. When, in this specification, a range is given as "(a first number) to (a second number)" or"(a first number) - (a second number)," this means a range whose lower limit is the first number and whose upper limit is the second number. For example, 25 to 100 mm means a range whose lower limit is 25mm, and whose upper limit is 100mm.

The term "area scan" is used herein to reference the act of looking at all parts of something in order to detect a feature by means of causing a part of the body to be traversed by a detector beam.

The present invention is related to a small, lightweight, self-contained, portable, ruggedized, head-mounted diagnostic tool for diagnosing conditions consistent with the existence of blockage in a patient's cranial blood vessels, including large vessel occlusions. Methods of diagnosing conditions consistent with the existence of blockage in a patient's cranial blood vessels, including large vessel occlusions, are also described. Multiple embodiments of the invention are described hereinafter with reference to the accompanying drawings. This invention may, however, be embodied in many different forms and should not be construed as limited to the embodiments set forth herein; rather, these embodiments are provided so that this disclosure will be thorough and complete and will fully convey the scope of the invention to those skilled in the art.

Described herein is a diagnostic apparatus 100 which utilizes near infrared imaging, and methods for their use to diagnose blockage in a patient's cranial blood vessels, including LVOs. According to an embodiment, referring to Fig. 1, the apparatus 100 is comprised of a headset 10. The headset 10 is adjustable so as fit the cranium of more than one patient.

Using the framework of a small head-harness that is transportable, field-expedient, and durable, a single pulse set using near-infrared energy is broadcast into the patient's brain allowing the apparatus to perform an area scan of the brain and detect and decipher cranial blood vessel blockage and LYO signal patterns. The interpretation of the pattern lies within the internal programming which produces a binary signal as to whether an LVO is suspected or not.

According to an embodiment, the headset 100 is comprised of an interior side 20 and an exterior side 30. According an embodiment, a scanning device is mounted on the interior side 20 of the headset 10. According to an embodiment, the scanning device is comprised of at least one array of transducers 40 which is mounted to the interior side 20 of the headset 10. According to an embodiment, the interior side 20 is comprised of a plurality of transducers 40, wherein the plurality of transducers 40 are arrayed. According to an embodiment, each transducer 40 is a non-focused near infrared transducer. According to an embodiment, each transducer array 40 is mounted to the interior side 20 of the headset 10. According to an embodiment, at least one mounted transducer 40 may be adjustably positioned on the interior side 20 of the headset 10.

According to an embodiment, the scanning device is comprised of at least two transducer arrays 40 where at least one transducer array 40 is configured to align with the left temple of a human patient; and at least one transducer array is configured to align with the right temple of a human patient. According to an embodiment, the apparatus 100 is further comprised of an electronic circuit 50. According to an embodiment, the electronic circuit 50 is operably connected to the headset 10. According to an embodiment, the electronic circuit controls each transducer 10. According to an embodiment, the electronic circuit 50 is comprised of a microcontroller 51 and memory 52 which comprise digitally encoded instructions in non-volatile memory for autonomously driving at least one diagnostic operation.

According to embodiments of the invention referring to Fig.'s 2B, 2C, 2D and 2E, the transducer array is configured to transmit energy into a patient's brain at varying angles of transmission 40. Reflected energy is detected by a sensor 60 which is proximately located or adjacent to at least one transducer 40.

Referred or reflected energy is detected by a sensor 60. According to an embodiment, the sensor 60 is proximately located or adjacent to at least one transducer 40. According to an embodiment the electronic circuit 50 is operably connected to the signal sensor 70. In an embodiment, the sensor is also operably connected to a signal interpreter 80. According to an embodiment, the signal sensor 70 and signal interpreter 80 are operably connected to the headset 10.

According to an embodiment of the invention, referring to Fig. 2A and Fig. 3, the digitally encoded instructions of electronic circuit 50 utilize the data collected by sensor 60 from a first side of the apparatus 100 (310) and data collected by sensor 60 from a second side of the apparatus 100 (320). Data is collected by the sensor 60 from a wide area of a patient's brain (Fig.'s 2C, 2D, and 2E), with the data set collected from a first portion of the patient's brain compared to the data set collected from a second portion of that patient's brain (310, 320) within the electronic circuit 50. In accordance with the present invention, a comparison is depicted in Fig. 3, comparing 330A to 330B, and 330C to 330D, wherein data collected is represented by nonspecific graphical waveforms. The programming of the digitally coded instructions in the electronic circuit 50 selects the most likely representative waveforms from the data collected (Fig. 3). If this process of comparing one data set to the corresponding data set identifies a deviation or variation in waveform, waveform set, or post-processed waveform set that exceeds a pre-determined threshold, a signal is transmitted to an output signal device 80.

According to an embodiment, a localizing device is mounted on the interior side 20 of the headset 10. An embodiment of the apparatus 100 utilizes the transducer or transducer array as the localizing device to identify and signal that a proper configuration of the harness has been achieved. In an embodiment the proper configuration is signaled through a visual output display 80. An embodiment of the apparatus 100 operably combines the scanning device and the localizing device.

An embodiment of the apparatus 100 is configured to use a separate transducer or transducer array as the localizing device using near infrared spectrum energy and decipher referred or reflected energy to identify and signal that a proper configuration of the harness has been achieved. The sensor array of this embodiment of the invention is comprised of near infrared imaging transmitters and receivers. In an embodiment, the proper configuration is signaled through a visual output display 80. An embodiment of the apparatus 100 operably combines the scanning device and the localizing device.

An embodiment of the apparatus 100 is configured to use a separate sector imaging phased array and decipher referred or reflected sound to identify and signal that a proper configuration of the harness has been achieved. The sensor array of this embodiment of the invention is comprised of a sector imaging phased array.

The apparatus 100 is configured to provide near infrared spectrum energy to a human patient and decipher referred or reflected energy to diagnose LVQ (610, 620, 630, 640). The sensor array of this embodiment of the invention is comprised of near infrared imaging transmitters and receivers.

According to an embodiment, the signal interpreter 70 examines and processes the detected energy signal patterns through deconvolution calculations. According to an embodiment, these patterns are represented through a visual output display 80 to signal whether an LVO is detected (440, 540, 640, 740).

According to an embodiment, the interior side 20 of the headset may attach to individually packaged, individual use, disposable pads that improve the transduction and sensing of signals.

Referring to the method described in Fig. 4, an apparatus employing scanning technology is mounted to the head of a patient (400, 410, 420) so that the scanner's transmitters and receivers (40 and 60) are situated adjacent to the temples of a patient's head. Proper contact between the scanning device and the patient's cranium is ensured using an appropriate insertional pad. A single pulse set is broadcast into the patient's brain allowing the apparatus to perform an area scan of the brain 430. Data is generated using reflected waves produced by each single pulse within a set which are collected by the receivers of the scanning apparatus 440. The status of blood flow in the patient's brain is analyzed by comparing the data collected from a first portion of the patient's brain to that collected from a second portion of the patient's brain 450. A diagnosis is developed based upon the analysis described (Fig. 3) with feedback provided to users of the head-mounted scanning apparatus 460 by way of the signal output device 80.

Referring to the method described in Fig. 5, an apparatus employing transcranial ultrasound scanning technology is mounted to the head of a patient (500, 510, 520) so that the scanner's transmitters and receivers (40 and 60) are situated adjacent to the temples of a patient's head. Proper contact between the scanning device and the patient's cranium is ensured using an appropriate insertional pad. A single pulse set is broadcast into the patient's brain allowing the apparatus to perform an area scan of the brain 530. Data is generated using reflected waves produced by each single pulse within a set which are collected by the receivers of the scanning apparatus 540. The status of blood flow' in the patient's brain is analyzed by comparing the data collected from a first portion of the patient's brain to that collected from a second portion of the patient's brain 550. A diagnosis is developed based upon the analysis described (Fig. 3) with feedback provided to users of the head-mounted scanning apparatus 560 by way of the signal output device 80.

Referring to the method described in Fig. 6, an apparatus employing near infrared scanning technology is mounted to the head of a patient (600, 610 620) so that the scanner's transmitters and receivers (40 and 60) are situated adjacent to the temples of a patient's head. Proper contact between the scanning device and the patient's cranium is ensured using an appropriate insertional pad. A single pulse set is broadcast into the patient's brain allowing the apparatus to perform an area scan of the brain 630, and generating a data set using reflected waves collected by the receivers of the scanning apparatus 640. The status of blood flow in the patient's brain is analyzed by comparing the data set collected from a first portion of the patient's brain to a data set collected from a second portion of the patient's brain 650. A diagnosis is developed based upon the analysis described (Fig. 3) with feedback provided to users of the head-mounted scanning apparatus 660 by way of the signal output device 80.

Referring to the method described in Fig. 7, an apparatus employing transcranial ultrasound scanning technology is mounted to the head of a patient (700 710, 720) so that the scanner's transmitters and receivers (40 and 60) are situated adjacent to the temples of a patient's head. Proper contact between the scanning device and the patient's cranium is ensured using an appropriate insertional pad. A single pulse set is broadcast into the patient's brain allowing the apparatus to perform an area scan of the brain 730 and generating a data set using reflected waves collected by the receivers of the scanning apparatus 740. The status of blood flow in the patient's brain is analyzed by comparing the data set collected from a first portion of the patient's brain to a data set collected from a second portion of the patient's brain 750. A diagnosis is developed based upon the analysis described (Fig. 3) with feedback provided to users of the head-mounted scanning apparatus 760 by way of the signal output device 80. If conditions consistent with large vessel occlusion are found to exist, treatment is initiated by targeting the impacted area, applying and maintaining focused ultrasound energy on the suspected LVO within the patient's brain 770.

## Claims

1. A head-mounted diagnostic tool (100) for diagnosing conditions consistent with the existence of blockage of a patient's cranial blood vessels, such as large vessel occlusions, comprising:
a. a power source;
b. at least one scanning device (40) for scanning a patient's brain comprising a transducer array incorporating a near-infrared imager transducer array and a receiver array, wherein the scanning device is stationary relative to the patient's cranium;
c. a data processing device (50) for analyzing and interpreting results generated by the scanning device;
d. an output device (80); and
e. an adjustable support (10) configured to: mount the scanning device the data processing device, and the output device to the cranium of a patient while the scanning operation is underway; focus the energy generated by the scanning device into the patient's cranium; and interconnect the power source, the scanning device, the data processing device, and the output device;
wherein the near-infrared imager transducer array is configured to transmit energy to a first portion and a second portion of the patient's brain;
the receiver array is configured to receive reflected energy from each of the first and second portions of the patient's brain;
the data processing device is configured to generate data sets using the reflected energy from each of the first and second portions of the patient's brain, and compare the data set collected from the first portion of the patient's brain to the data set collected from the second portion of the patient's brain, wherein the data processing device is configured to represent the data sets by non-specific graphical waveforms, by selecting the most likely representative waveforms from the data collected;
the data processing device is further configured to transmit a signal to the output device if the comparison of the data sets identifies a deviation or variation in waveform, waveform set or post-processed waveform set that exceeds a pre-determined threshold; and
the output device is configured to generate an output based on the results generated by data processing device.

2. The apparatus of claim 1 wherein the number of scanning devices is two or more.

## Patentansprüche

1. Ein am Kopf befestigtes Diagnosewerkzeug (100) zum Diagnostizieren von Zuständen, die mit dem Bestehen einer Obstruktion kranialer Blutgefäße eines Patienten, wie etwa Verschlüssen großer Gefäße, übereinstimmend sind, beinhaltend:
a. eine Stromquelle;
b. mindestens eine Scanvorrichtung (40) zum Scannen des Gehirns eines Patienten, beinhaltend ein Wandlerarray, das ein Nahinfrarotbildgeber-Wandlerarray und ein Empfängerarray enthält, wobei die Scanvorrichtung relativ zu dem Kranium des Patienten stationär ist;
c. eine Datenverarbeitungsvorrichtung (50) zum Analysieren und Interpretieren der von der Scanvorrichtung erzeugten Ergebnisse;
d. eine Ausgabevorrichtung (80); und
e. einen anpassbaren Träger (10), der für Folgendes konfiguriert ist: Befestigen der Scanvorrichtung, der Datenverarbeitungsvorrichtung und der Ausgabevorrichtung an dem Kranium eines Patienten, während der Scanvorgang abläuft; Fokussieren der von der Scanvorrichtung erzeugten Energie in das Kranium des Patienten; und Verbinden der Stromquelle, der Scanvorrichtung, der Datenverarbeitungsvorrichtung und der Ausgabevorrichtung miteinander;
wobei das Nahinfrarotbildgeber-Wandlerarray konfiguriert ist, um Energie auf einen ersten Abschnitt und einen zweiten Abschnitt des Gehirns des Patienten zu übertragen; das Empfängerarray konfiguriert ist, um von jedem von dem ersten und dem zweiten Abschnitt des Gehirns des Patienten reflektierte Energie zu empfangen;
die Datenverarbeitungsvorrichtung konfiguriert ist, um unter Verwendung der reflektierten Energie von jedem von dem ersten und dem zweiten Abschnitt des Gehirns des Patienten Datensätze zu erzeugen und den von dem ersten Abschnitt des Gehirns des Patienten gesammelten Datensatz mit dem von dem zweiten Abschnitt des Gehirns des Patienten gesammelten Datensatz zu vergleichen, wobei die Datenverarbeitungsvorrichtung konfiguriert ist, um die Datensätze durch das Auswählen der am wahrscheinlichsten repräsentativen Wellenformen aus den gesammelten Daten durch nichtspezifische graphische Wellenformen darzustellen;
die Datenverarbeitungsvorrichtung ferner konfiguriert ist, an die Ausgabevorrichtung ein Signal zu übertragen, wenn der Vergleich der Datensätze eine Abweichung oder Variation einer Wellenform, eines Wellenformsatzes oder eines nachbearbeiteten Wellenformsatzes identifiziert, die einen vorgegebenen Schwellenwert überschreitet; und
die Ausgabevorrichtung konfiguriert ist, um auf der Basis der von der Datenverarbeitungsvorrichtung erzeugten Ergebnisse eine Ausgabe zu erzeugen.

2. Einrichtung gemäß Anspruch 1, wobei die Anzahl der Scanvorrichtungen zwei oder mehr beträgt.

## Revendications

1. Un outil de diagnostic monté sur la tête (100) destiné à diagnostiquer des problèmes de santé compatibles avec l'existence d'un blocage de vaisseaux sanguins crâniens d'un patient, telles des occlusions de gros vaisseaux, comprenant :
a. une source d'alimentation ;
b. au moins un dispositif de balayage (40) destiné à balayer le cerveau d'un patient comprenant un groupement de transducteurs incorporant un groupement de transducteurs imageurs dans le proche infrarouge et un groupement de récepteurs, le dispositif de balayage étant stationnaire par rapport au crâne du patient ;
c. un dispositif de traitement de données (50) destiné à analyser et interpréter des résultats générés par le dispositif de balayage ;
d. un dispositif de sortie (80) ; et
e. un support réglable (10) configuré pour : monter le dispositif de balayage, le dispositif de traitement de données, et le dispositif de sortie au crâne d'un patient pendant que l'opération de balayage est en cours ; focaliser l'énergie générée par le dispositif de balayage dans le crâne du patient ; et connecter entre eux la source d'alimentation, le dispositif de balayage, le dispositif de traitement de données, et le dispositif de sortie ;
dans lequel le groupement de transducteurs imageurs dans le proche infrarouge est configuré pour émettre de l'énergie à destination d'une première portion et d'une deuxième portion du cerveau du patient ;
le groupement de récepteurs est configuré pour recevoir de l'énergie réfléchie provenant de chacune des première et deuxième portions du cerveau du patient ;
le dispositif de traitement de données est configuré pour générer des ensembles de données en utilisant l'énergie réfléchie provenant de chacune des première et deuxième portions du cerveau du patient, et comparer l'ensemble de données recueilli auprès de la première portion du cerveau du patient à l'ensemble de données recueilli auprès de la deuxième portion du cerveau du patient, le dispositif de traitement de données étant configuré pour représenter les ensembles de données par formes d'onde graphiques non spécifiques, par sélection des formes d'onde les plus probablement représentatives parmi les données recueillies ;
le dispositif de traitement de données est configuré en outre pour émettre un signal à destination du dispositif de sortie si la comparaison des ensembles de données identifie un écart ou une variation dans une forme d'onde, un ensemble de formes d'onde ou un ensemble de formes d'onde post-traité qui dépasse un seuil prédéterminé ; et
le dispositif de sortie est configuré pour générer une sortie sur la base des résultats générés par le dispositif de traitement de données.

2. L'appareil de la revendication 1 dans lequel le nombre de dispositifs de balayage est deux ou plus.
